(19) Europäisches Patentamt / European Patent Office / Office européen des brevets

(11) **EP 4 375 663 B1**

(12) **FASCICULE DE BREVET EUROPEEN**

(45) Date de publication et mention de la délivrance du brevet:
**27.08.2025 Bulletin 2025/35**

(21) Numéro de dépôt: **23211842.2**

(22) Date de dépôt: **23.11.2023**

(51) Classification Internationale des Brevets (IPC):
***G01N 33/24*** *(2006.01)* ***G01S 13/88*** *(2006.01)*
***G01V 3/12*** *(2006.01)* ***G01S 7/41*** *(2006.01)*

(52) Classification Coopérative des Brevets (CPC):
**G01N 33/24; G01S 7/411; G01S 13/885; G01V 3/12**

(54) **MÉTHODE ET DISPOSITIF POUR DÉTERMINER LA PERMITTIVITÉ RELATIVE D'UN MATÉRIAU À L'AIDE D'UN RADAR À PÉNÉTRATION DE SOL**

VERFAHREN UND VORRICHTUNG ZUR BESTIMMUNG DER RELATIVEN PERMITTIVITÄT EINES MATERIALS MITTELS EINES BODENDURCHDRINGENDEN RADARS

METHOD AND APPARATUS FOR DETERMINING THE RELATIVE PERMITTIVITY OF A MATERIAL USING A GROUND PENETRATING RADAR

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **28.11.2022 FR 2212411**

(43) Date de publication de la demande:
**29.05.2024 Bulletin 2024/22**

(73) Titulaire: **Commissariat à l'Energie Atomique et aux Energies Alternatives**
**75015 Paris (FR)**

(72) Inventeurs:
- **MAKHOUL, Gloria**
  **38054 GRENOBLE CEDEX 09 (FR)**
- **D'ERRICO, Raffaele**
  **38054 GRENOBLE CEDEX 09 (FR)**

(74) Mandataire: **Atout PI Laplace**
**Immeuble Up On**
**25 Boulevard Romain Rolland**
**CS 40072**
**75685 Paris Cedex 14 (FR)**

(56) Documents cités:
- **WALIA A ET AL: "Reviewing methods for determination of Dielectric Constant required to Calibrate GPR Study for Asphalt Layers", IOP CONFERENCE SERIES: MATERIALS SCIENCE AND ENGINEERING, vol. 1075, 8 March 2021 (2021-03-08), GB, pages 012026, XP093059141, ISSN: 1757-8981, DOI: 10.1088/1757-899X/1075/1/012026**
- **UMBERTO SPAGNOLINI: "Permittivity Measurements of Multilayered Media With Monostatic Pulse Radar", IEEE TRANSACTIONS ON GEOSCIENCE AND REMOTE SENSING, IEEE, USA, vol. 35, no. 2, March 1997 (1997-03-01), XP011020837, ISSN: 0196-2892**
- **WANG HUI ET AL: "Deep-Learning-Based Method for Estimating Permittivity of Ground-Penetrating Radar Targets", REMOTE SENSING, vol. 14, no. 17, 31 August 2022 (2022-08-31), pages 4293, XP093059139, DOI: 10.3390/rs14174293**
- **ZHANG WENJI ET AL: "MIMO Ground Penetrating Radar Imaging Through Multilayered Subsurface Using Total Variation Minimization", IEEE TRANSACTIONS ON GEOSCIENCE AND REMOTE SENSING, IEEE, USA, vol. 57, no. 4, 25 March 2019 (2019-03-25), pages 2107 - 2115, XP011716749, ISSN: 0196-2892, [retrieved on 20190325], DOI: 10.1109/TGRS.2018.2871463**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 4 375 663 B1

## Description

L'invention concerne le domaine des radars à pénétration de sol et plus précisément des dispositifs et méthodes non destructives permettant de caractériser la composition du sol ou plus généralement d'un matériau.

L'invention porte plus précisément sur une méthode et un dispositif permettant de déterminer les caractéristiques diélectriques du sol, autrement dit les permittivités relatives et les épaisseurs des différentes couches qui constituent le sol. Ces informations permettent ensuite d'améliorer les techniques de détection radar afin de localiser et identifier des cibles souterraines telles que des tuyaux, canalisations ou encore des mines ou plus généralement tout type d'objet enterré dans le sol ou dans un matériau quelconque.

Les méthodes de détection de cibles enterrées à l'aide d'un radar à pénétration de sol sont généralement basées sur des algorithmes qui nécessitent, dans leurs paramètres, une estimation précise des permittivités relatives des différentes couches du sol. En général, ces informations ne sont pas connues ou sont estimées grossièrement sous la forme d'une valeur moyenne pour l'ensemble de la structure du sol. Ces approximations peuvent augmenter les incertitudes sur les résultats de détection et caractérisation des cibles souterraines.

Il y a donc un besoin pour une méthode permettant d'estimer précisément les permittivités relatives des différentes couches du sol ainsi que leurs épaisseurs afin d'utiliser ultérieurement ces informations pour améliorer les algorithmes de détection de cibles au moyen de radars à pénétration de sol.

Dans la suite du texte, l'invention est décrite dans le contexte de la détection de cibles enterrées dans le sol mais l'invention s'applique plus généralement à des cibles enterrées dans un matériau quelconque qui peut être différent du sol. L'invention s'applique également à l'imagerie médicale par microonde pour détecter, par exemple, des veines sous la peau ou encore au contrôle non destructif par microonde pour estimer l'épaisseur de couches de béton d'une structure industrielle.

Plusieurs méthodes d'estimation de la permittivité ont été proposées au fil des années à l'aide de données issues de radars à pénétration de sol ou « Ground Penetration Radar » GPR en anglais, par exemple les références [1-2]. La plupart de ces techniques sont analogues aux approches d'analyse de vitesse utilisées dans le traitement des données sismiques tel que décrit dans [3]. Chacune de ces méthodes dépend, dans une certaine mesure, de la comparaison de mesures connues avec des données acquises par un radar à pénétration de sol GPR. Les deux méthodes de l'art antérieur les plus utilisées sont l'ajustement de courbe hyperbole et la migration.

La première méthode connue est basée sur l'ajustement d'une courbe hyperbole. Lorsque l'antenne du radar GPR est déplacée sur la surface du sol et que l'onde pénétrante rencontre des cibles enterrées ou des interfaces entre différentes couches du sol, des hyperboles peuvent être formées par les temps de parcours des ondes réfléchies sur la distance parcourue par le radar. La forme de chaque hyperbole est donnée principalement à partir de deux éléments : la conception du radar, en particulier la disposition géométrique des antennes, et les propriétés diélectriques du milieu de propagation. La forme du système radar est statique mais la portion de cible illuminée par le radar augmente avec la profondeur, et le contour de la courbe change alors avec la profondeur. La forme de chaque hyperbole individuelle est donc fonction de la profondeur et des propriétés diélectriques du milieu. Connaissant la profondeur cible, on peut estimer les propriétés diélectriques moyennes du milieu en ajustant une courbe numérique au contour de l'hyperbole dans les données tel que décrit dans la référence [4]. Il s'agit d'une méthode très courante d'étalonnage de données GPR ; néanmoins, cette méthode peut présenter deux défauts potentiels. Le premier inconvénient est que les données acquises doivent contenir plusieurs hyperboles suffisamment claires pour des profondeurs cibles connues pour pouvoir exploiter cette méthode. L'autre problème potentiel est qu'il existe généralement une petite plage de valeurs qui correspond au même contour d'hyperbole. Ce dernier problème peut cependant être affiné avec plus de précision en utilisant une technique mathématique connue sous le nom de migration et décrit dans la référence [5].

La migration est une forme de traitement mathématique dont le but principal est de définir plus précisément les objets linéaires. Correctement appliqué, le processus réduit les hyperboles en un point des données acquises par le radar. Pour les objets linéaires, cela rend leur position et leur profondeur beaucoup plus faciles à définir car il n'est plus nécessaire d'exploiter l'hyperbole complète. Il existe une grande variété de méthodes de migration différentes parmi lesquelles celle décrite dans la référence [5]. Le principal paramètre requis pour réduire l'hyperbole à un point correspond aux propriétés diélectriques du sol tel qu'enseigné dans la référence [6]. Cela fait du processus de migration un moyen d'augmenter la précision de l'ajustement de la courbe puisque le processus ne fonctionne correctement que si la vitesse de transmission au sol appliquée est précise.

Un aperçu des méthodes établies de détermination de la permittivité relative dans le contexte des radars à pénétration le sol est donné dans la référence [9].

De manière générale, les méthodes de l'art antérieur utilisent une estimation moyenne de la permittivité relative d'un matériau pour une profondeur donnée.

Un inconvénient de ces méthodes est leur relative imprécision lorsque le matériau, en particulier le sol, est composé de plusieurs couches de permittivités différentes.

L'invention propose une nouvelle méthode permettant d'estimer précisément les permittivités relatives et les

épaisseurs de chaque couche d'un matériau tel que le sol.

La méthode est basée sur des acquisitions de mesures réalisées par un radar à pénétration de sol muni de plusieurs antennes en émission et plusieurs antennes en réception (radar MIMO « Multiple Input Multiple Output »).

La méthode proposée se base sur un algorithme particulier qui vise à estimer les couples (retard, permittivité relative) les plus probables qui correspondent aux différentes couches composant la structure du sol.

L'invention a pour objet une méthode de détermination de la permittivité relative d'un matériau comprenant les étapes de :

- Acquérir un ensemble de mesures à l'aide d'un radar à pénétration de sol muni de NTx antennes en émission et NRx antennes en réception disposées dans un plan parallèle à la surface du matériau, avec NRx et NTx deux entiers positifs au moins égaux à deux, l'ensemble de mesures étant constitué de réponses impulsionnelles pour chaque couple constitué d'une antenne en émission et d'une antenne en réception,
- Extraire de chaque réponse impulsionnelle, les composantes relatives à des trajets multiples du signal radar, sous la forme d'un vecteur donnant l'amplitude du signal en fonction de différentes valeurs de retards correspondant à différents trajets,
- Estimer, pour chaque antenne en émission, une coordonnée, dans le plan du radar, de chaque cible associée à un trajet en recherchant la coordonnée de l'antenne en réception qui maximise la réponse impulsionnelle,
- Calculer, pour plusieurs hypothèses de valeurs de permittivité relative du matériau, une estimée d'un retard du signal radar à partir d'un modèle de courbe hyperbolique défini par les caractéristiques du radar et les coordonnées de la cible associée à un trajet du signal,
- Rechercher, dans l'ensemble des composantes extraites des réponses impulsionnelles, pour chaque couple constitué d'une antenne en émission et d'une antenne en réception, au moins une amplitude associée à un retard le plus proche de l'estimée d'un retard calculé à l'étape précédente,
- Calculer, pour chaque antenne en émission, un coefficient de corrélation desdites amplitudes sur l'ensemble des antennes en réception,
- Itérer les trois étapes précédentes pour plusieurs valeurs de permittivité relative du matériau,
- Itérer les quatre étapes précédentes pour toutes les valeurs de retard correspondant à des trajets multiples du signal radar pour obtenir NTx matrices de corrélation donnant, pour chaque couple de valeur de permittivité relative du sol et valeur de retard, une valeur de coefficient de corrélation,
- Eliminer les valeurs des matrices de corrélation pour lesquelles le coefficient de corrélation est inférieur à un seuil de détection prédéterminé,
- Filtrer les valeurs restantes pour ne conserver qu'un vecteur contenant un ensemble de valeurs de retard toutes différentes et une valeur associée de permittivité relative du sol,
- En déduire, pour chaque couche du sol, une estimée de sa permittivité relative et de son épaisseur.

Selon un aspect particulier de l'invention, l'étape d'extraire de chaque réponse impulsionnelle, les composantes relatives à des trajets multiples du signal radar est réalisée au moyen d'un algorithme par haute résolution.

Selon un aspect particulier de l'invention, l'étape de calcul d'une estimée t d'un retard du signal radar est réalisée au moyen de l'équation hyperbolique suivante :

$$t = \frac{1}{v_g}\left[\sqrt{\left(x_{Tx_j} - x_{c_p}\right)^2 + \left(\frac{\Delta y}{2}\right)^2 + \left(\frac{v_g \tau_p}{2}\right)^2} + \sqrt{\left(x_{Rx_i} - x_{c_p}\right)^2 + \left(\frac{\Delta y}{2}\right)^2 + \left(\frac{v_g \tau_p}{2}\right)^2}\right]$$

Avec $x_{c_p}$ la coordonnée de la cible estimée, $x_{Tx_j}$ la coordonnée de chaque antenne en émission, $x_{Rx_i}$ la coordonnée de chaque antenne en réception, $\Delta y$ la distance entre une antenne en émission et une antenne en réception, $v_g$ la vitesse moyenne de propagation du signal dans le matériau, $\tau_p$ la valeur d'un retard estimée.

Selon un aspect particulier de l'invention, l'étape de rechercher au moins une amplitude associée à un retard le plus proche de l'estimée d'un retard calculé à l'étape précédente est réalisée en :

- Recherchant, pour chaque couple constitué d'une antenne en émission et d'une antenne en réception, l'amplitude $a_{ip}$ associée au retard le plus proche de l'estimée d'un retard calculé,

- Définir une fenêtre temporelle de N+1 échantillons, N étant un entier positif au moins égal à 1, la fenêtre étant centrée sur l'amplitude sélectionnée à l'étape précédente et sélectionner N/2 valeurs d'amplitudes correspondants aux valeurs de retard comprises dans la fenêtre temporelle.

Selon un aspect particulier de l'invention, le coefficient de corrélation est calculé au moyen de la relation suivante :

$$R(t,\varepsilon_r) = \frac{\sum_{p_j=p-N/2}^{p+N/2}\left[\sum_{i=1}^{NRx} a_{ip_j}\right]^2}{M\sum_{p_j=p-N/2}^{p+N/2}\sum_{i=1}^{NRx} a_{ip_j}^2}$$

avec $a_{ip}$ l'amplitude associée au retard le plus proche de l'estimée d'un retard calculé, p est l'indice dudit retard et $a_{ipj}$ est l'amplitude correspondant à l'indice temporel $p_j$ dans la dite fenêtre temporelle, $\varepsilon_r$ est l'hypothèse de valeur de permittivité relative.

Selon un aspect particulier de l'invention, la valeur du seuil est comprise dans l'intervalle ] 0.5 ; 1 [

Selon un aspect particulier de l'invention, l'étape de filtrer les valeurs restantes comporte les sous étapes de :

- Sélectionner les couples qui présentent sensiblement les mêmes valeurs de retard pour toutes les antennes en émission,

- Ordonner les couples selon la valeur des retards,

- Lorsque plusieurs valeurs de permittivité relative consécutives sont sensiblement égales, conserver uniquement le couple ayant la valeur de retard la plus élevée.

Selon un aspect particulier de l'invention, lorsque plusieurs couples présentent sensiblement les mêmes valeurs de retard pour toutes les antennes en émission mais des valeurs de permittivité relative différentes, on conserve uniquement la valeur de permittivité relative qui maximise le coefficient de corrélation.

Selon un aspect particulier de l'invention, l'étape de déduire, pour chaque couche du matériau, une estimée de sa permittivité relative et de son épaisseur comporte les sous étapes de :

- Estimer la profondeur apparente de chaque couche à partir de chaque couple de valeurs de retard et de permittivité relative,

- Déterminer itérativement la permittivité relative de chaque couche en appliquant l'équation de Dix aux valeurs de retard et de vitesse de propagation, dérivée de la valeur de permittivité relative,

- Déterminer l'épaisseur de chaque couche à partir des valeurs de retard et de vitesse de propagation.

Selon un aspect particulier de l'invention, au cours de l'étape de déterminer itérativement la permittivité relative de chaque couche, lorsque deux trajets consécutifs conduisent à obtenir une vitesse de propagation imaginaire, le trajet, parmi les deux trajets, ayant le facteur de corrélation le plus faible est éliminé.

Selon un aspect particulier de l'invention, le matériau est multi-couches et la méthode comprend une phase préalable d'estimation de la permittivité relative de la première couche du matériau pour laquelle le modèle de courbe hyperbolique pour la première couche du matériau est un modèle asymptotique linéaire et la phase préalable comprend :

- une étape de détermination du trajet correspondant à la première couche comme celui qui maximise le coefficient de corrélation,

- la sélection de la permittivité relative associée audit trajet comme correspondant à la première couche,

- le calcul de l'épaisseur de la première couche à partir des valeurs de retard et permittivité relative associées au trajet.

Selon un aspect particulier de l'invention, le seuil de détection utilisé pour la première couche lors de la phase préalable a une valeur inférieure au seuil de détection utilisé pour les couches ultérieures.
Selon un aspect particulier de l'invention, le matériau est le sol.
L'invention a aussi pour objet un dispositif pour déterminer la permittivité relative d'un matériau comprenant un radar à pénétration de sol muni de NTx antennes en émission et NRx antennes en réception et une unité de traitement configurés pour mettre en œuvre les étapes de la méthode selon l'invention.
L'invention a aussi pour objet un programme d'ordinateur comprenant des instructions de code qui conduisent le dispositif selon l'invention à exécuter les étapes de la méthode selon l'invention ainsi qu'un support lisible par ordinateur, sur lequel est enregistré le programme d'ordinateur selon l'invention.
D'autres caractéristiques et avantages de la présente invention apparaîtront mieux à la lecture de la description qui suit en relation aux dessins annexés suivants.

[Fig. 1] représente un organigramme détaillant les étapes de mise en œuvre d'une méthode de détermination de la permittivité du sol selon un mode de réalisation de l'invention,

[Fig. 2a] représente un premier schéma d'un radar à pénétration de sol multi antennes,

[Fig. 2b] représente un second schéma d'un radar à pénétration de sol multi antennes,

[Fig. 3a] représente un exemple d'une matrice de réponses impulsionnelles mesurée pour une antenne d'émission,

[Fig. 3b] illustre une étape de détection de trajets multiples dans la matrice de la figure 3a,

[Fig. 3c] représente un exemple d'une matrice de corrélation selon une étape de la méthode selon l'invention,

[Fig. 4] représente un schéma simplifié d'un dispositif configuré pour mettre en œuvre l'invention.

La figure 1 représente, sur un organigramme, les étapes de mise en œuvre d'une méthode de détermination de caractéristiques diélectriques du sol selon un premier mode de réalisation de l'invention.
Ce premier mode de réalisation s'applique avantageusement lorsque l'épaisseur de la première couche du sol est suffisamment grande devant la résolution temporelle du radar qui dépend de sa fréquence porteuse de fonctionnement.
La première étape 101 consiste à acquérir un ensemble de mesures au moyen d'un radar à pénétration de sol ayant NTx antennes fonctionnant en émission et NRx antennes fonctionnant en réception, avec NTx,NRx des entiers positifs au moins égaux à 2. La figure 2a schématise un exemple de radar RPS ayant cinq antennes en émission et quatre antennes en réception et se déplaçant sur la surface du sol S en réalisant plusieurs acquisitions temporelles successives.
La figure 2b représente un autre exemple de radar à pénétration de sol RPS en vue de dessus dans un plan (x,y) parallèle au plan du sol. Les NRx antennes en réception sont chacune disposées à une distance fixe $\Delta y$ des NTx antennes en émission. Chaque antenne en émission présente une coordonnée $x_{Rxi}$ selon l'axe x et est séparée d'une distance $\Delta y$ avec une antenne en réception correspondante selon l'axe y.
Sans sortir du cadre de l'invention, d'autres géométries de disposition des antennes du radar sont envisageables. Dans tous les cas, le radar RPS est disposé à proximité du sol pour fonctionner en champ proche.
L'étape 101 d'acquisition de mesures radars consiste ainsi à déplacer le radar RPS sur la surface du sol S à analyser et à mesurer, pour chaque couple associant une antenne en émission et une antenne en réception, une réponse impulsionnelle du signal radar $h(Rx_i, Tx_j, \tau)$, où $Rx_i$ désigne une antenne en réception, $Tx_j$ désigne une antenne en émission et $\tau$ désigne le retard temporel. Autrement dit la réponse impulsionnelle donne les valeurs d'amplitude en fonction du retard $\tau$.
Les réponses impulsionnelles peuvent être organisées sous la forme d'un nombre NTx de vecteurs de dimension NRx.
A l'étape 102, on applique ensuite aux réponses impulsionnelles une méthode d'extraction des composantes relatives aux trajets multiples. Par exemple, la méthode utilisée est la méthode CLEAN ou une méthode de haute résolution ou toute autre méthode appropriée.
L'étape 102 a pour but de détecter des trajets multiples du signal qui correspondent à des réflexions sur des cibles enterrées. Chaque trajet est caractérisé par un retard $\tau_p$ et une amplitude $|h(Rx_i, Tx_j, \tau_p)|$.
Dans un mode de réalisation particulier de l'étape 102, la méthode CLEAN est appliquée au moyen d'un filtre rectangle qui est défini par un nombre d'échantillons dont les amplitudes sont forcées à zéro autour du maximum local

détecté. Une approche dite par largeur de faisceau ou « beamwidth filter » consiste à rechercher les minima locaux les plus proches autour du maximum local détecté, puis à forcer l'échantillon formant l'impulsion détectée à zéro. Des exemples de réalisation de la méthode CLEAN sont donnés dans les références [7] et [8].

A l'issue de l'étape 102, on obtient pour chaque antenne d'émission $Tx_j$ une matrice 2D de dimension NP par NRx qui donne les valeurs des amplitudes de chaque trajet $\tau_p$ détecté pour chaque antenne de réception $Rx_i$, avec NP le nombre de trajets détectés.

Les étapes 103,104 suivantes consistent à modéliser chaque trajet du signal au moyen d'une courbe hyperbolique qui dépend de la géométrie du radar, des caractéristiques diélectriques du sol et de la position des cibles sur lesquelles sont réfléchis les trajets du signal.

Un exemple de trajet modélisé par une courbe hyperbolique est donné par l'équation (1) suivante :

$$t = \frac{1}{v_g}\left[\sqrt{\left(x_{Tx_j} - x_{c_p}\right)^2 + \left(\frac{\Delta y}{2}\right)^2 + \left(\frac{v_g \tau_p}{2}\right)^2} + \sqrt{\left(x_{Rx_i} - x_{c_p}\right)^2 + \left(\frac{\Delta y}{2}\right)^2 + \left(\frac{v_g \tau_p}{2}\right)^2}\right] \quad (1)$$

$v_g$ est la vitesse de propagation du signal vers un point d'une couche du sol qui est liée à la permittivité relative moyenne $\varepsilon_r$ du milieu constituant la couche du sol par l'équation (2) :

$$v_g = \frac{c}{\sqrt{\varepsilon_r}} \quad (2)$$

c est la célérité de la lumière en espace libre.
$\Delta y$ est la distance, selon l'axe y de la figure 2b, entre une antenne d'émission et une antenne de réception
$\tau_p$ est le retard associé au trajet d'indice p
$x_{c_p}$ est l'abscisse, selon l'axe x de la figure 2b, de la cible associée au trajet d'indice p
$x_{Tx_j}$ et $X_{Rx_i}$ sont les abscisses respectives des antennes en émission et en réception.

Sans sortir du cadre de l'invention, l'équation (1) qui modélise une courbe hyperbolique peut être adaptée selon la géométrie du radar.

A l'étape 103, on détermine tout d'abord une estimation de la coordonnée $x_{c_p}$ de la cible en recherchant, pour chaque antenne en émission et chaque trajet d'indice p, la coordonnée de l'antenne en réception qui maximise la réponse impulsionnelle déterminée à l'étape 102 :

$$x_{cp} = \text{argmax}_{x_{Rxi}} \left|h\left(Rx_i, Tx_j, \tau_p\right)\right| \quad (3)$$

Ensuite, à l'étape 104, on applique l'équation (1) pour estimer le retard t modélisé à partir d'une courbe hyperbolique, pour différentes hypothèses de valeurs de permittivité relative $\varepsilon_r$ et pour toutes les antennes en réception.

A l'étape 105, on recherche ensuite, dans les réponses impulsionnelles, pour chaque antenne de réception, l'amplitude $a_{ip}$ associée au retard $\tau_p$ qui est le plus proche du retard t calculé à l'étape 104. Autrement dit, on retient l'indice p qui minimise la différence entre t et $\tau_p$ parmi l'ensemble des retards détectés pour une antenne de réception.

Autrement dit, en utilisant $\tau_p$ et $x_{c_p}$ pour une permittivité donnée l'étape 104 consiste à calculer un vecteur de retards t de dimension égale au nombre d'antennes en réception NRx. Ce vecteur a la forme de l'hyperbole formée par une cible de retard $\tau_p$ et $x_{c_p}$.

Ensuite, il convient de rechercher cette hyperbole dans les données de mesure. Pour cela, on cherche les amplitudes correspondants au vecteur t ([1xNRx] = [t1 t2 t3 tNRx]) dans les données $h(x_{Txi}, x_{Rxj}, t)$.

Parfois les valeurs de t ne se retrouvent pas à l'identique dans les mesures. Dans ce cas, deux options sont possibles. Une première option consiste à rechercher le retard $\tau_p$ le plus proche de t et sélectionner l'amplitude correspondante.

Une autre option consiste à réaliser une interpolation sur l'amplitude obtenue pour le retard $\tau_p$ afin d'estimer la valeur de l'amplitude correspondant au retard t.

Dans un mode de réalisation particulier de l'étape 105, plusieurs amplitudes sont retenues dans une fenêtre temporelle de dimension N+1 autour de l'amplitude $a_{ip}$ associée au retard $\tau_p$ qui est le plus proche du retard t.

A l'étape 106, on calcule ensuite un coefficient de corrélation encore appelé métrique de ressemblance qui vise à estimer le niveau de ressemblance entre les amplitudes déterminées à l'étape 105 sur l'ensemble des antennes de réception. L'objectif de l'étape 105 est d'identifier si les amplitudes du trajet retenu sont sensiblement identiques pour toutes les antennes de réception ce qui doit normalement être le cas si le trajet est correctement estimé.

Le coefficient de corrélation est par exemple déterminé au moyen de la relation suivante :

$$R(t,\varepsilon_r) = \frac{\sum_{p_j=p-N/2}^{p+N/2}\left[\sum_{i=1}^{NRx} a_{ip_j}\right]^2}{M\sum_{p_j=p-N/2}^{p+N/2}\sum_{i=1}^{NRx} a_{ip_j}^2} \quad (4)$$

M est un facteur de normalisation dimensionné de telle sorte que le coefficient de corrélation vaut 1 lorsque toutes les amplitudes sont égales et tend vers 0 lorsqu'elles sont fortement différentes.

Comme indiqué, dans un mode de réalisation, N est pris égal à 0 et la relation (4) se simplifie en :

$$R(t,\varepsilon_r) = \frac{\left[\sum_{i=1}^{NRx} a_{ip_j}\right]^2}{M\sum_{i=1}^{NRx} a_{ip_j}^2}$$

Sans sortir du cadre de l'invention, d'autres métriques peuvent être utilisées pour calculer un coefficient de ressemblance.

Les étapes 104 à 106 sont itérées pour toutes les hypothèses de permittivité relative. Les étapes 103 à 106 sont itérées pour tous les indices de retard p.

A l'issue de ces étapes on obtient NTx matrices de corrélation encore appelés matrices de cohérence de dimensions NP par NE, où NP est le nombre de retards et NE le nombre d'hypothèses de permittivités relatives. Les valeurs d'une matrice de corrélation sont les coefficients de corrélation $R(t, \varepsilon_r)$ pour chaque couple retard, permittivité relative.

Ensuite à l'étape 107, on compare toutes les valeurs des NTx matrices de corrélation à un seuil de détection prédéterminé th1 dont la valeur est comprise dans l'intervalle ]0.5 ; 1[.

Seules les valeurs qui dépassent ce seuil sont conservées. Une valeur élevée du seuil th1 permet d'être plus sélectif afin d'éliminer un plus grand nombre de valeurs. A l'inverse, une valeur plus faible peut être choisie lorsque le modèle de courbe hyperbolique présente davantage d'incertitudes.

A l'issue de l'étape 107, on obtient une matrice 3D (délai, permittivité relative et coefficient de corrélation) de dimensions NP' par NTx par NE' ou NP' et NE ' correspondent respectivement aux nombres maximum de trajets et d'hypothèses de permittivité pour chaque antenne d'émission.

La matrice obtenue est de la forme suivante, où chaque colonne représente les évènements significatifs détectés par l'ensemble des antennes en réception pour une seule antenne d'émission.

$$A = \begin{pmatrix} \alpha_{11} & \cdots & \alpha_{1NTx} \\ \vdots & \ddots & \vdots \\ \alpha_{\mathrm{NP}'1} & \cdots & \alpha_{\mathrm{NP}'NTx} \end{pmatrix}$$

Avec $\alpha_{pi}$ est une matrice 2D de la forme :

$$\alpha_{pi} = \begin{pmatrix} \tau_{pi} & \varepsilon_{p1} & R(\varepsilon_{p1},\tau_{pi})_{p1} \\ \vdots & \ddots & \vdots \\ \tau_{pi} & \varepsilon_{pNE'} & R(\varepsilon_{pNE'},\tau_{pi})_{pNE'} \end{pmatrix}$$

La première colonne de cette matrice contient sur chaque entrée la même valeur de retard $\tau_{pi}$, la deuxième colonne contient les différentes valeurs retenues de permittivité relative et la troisième colonne contient les valeurs de coefficient de corrélation associées au couple retard, permittivité.

L'étape 108 consiste ensuite à filtrer les valeurs de la matrice A afin de ne conserver qu'un vecteur de couples retard, permittivité pour lequel toutes les valeurs de retard sont différentes.

Pour cela, l'étape 108 consiste tout d'abord à conserver les trajets multiples communs entre les différentes antennes d'émission. Chaque trajet est défini par un couple (retard, permittivité relative). Les trajets communs sont ceux qui présentent des retards proches en se basant sur un critère d'erreur donné. La différence entre deux retards est comparée en valeur absolue à un seuil d'erreur qui dépend du scénario visé et notamment de la vitesse de mouvement du

radar lors de son déplacement pendant l'étape d'acquisition 101 mais aussi de la taille du réseau d'antennes. Lorsque plusieurs trajets communs sont détectés ayant sensiblement la même valeur de retard mais des valeurs de permittivité relative différentes, le trajet qui présente le facteur de corrélation le plus élevé est conservé, les autres sont éliminés.

Après cette étape, les trajets sont triés par ordre croissant des valeurs des retards. Si deux trajets consécutifs présentent des permittivités relatives sensiblement égales, seul le trajet qui présente le retard le plus élevé est conservé, les autres sont supprimés.

En sortie de l'étape 108, on obtient donc un vecteur final dont la dimension correspond au nombre de couches du sol estimé et dont chaque composante correspond à un couple retard, permittivité relative.

L'étape 109 consiste en l'étape finale qui vise à estimer les permittivités relatives de chaque couche du sol et son épaisseur.

L'étape 109 consiste tout d'abord à calculer une profondeur apparente entre la surface du sol et le fond de chaque couche successive en utilisant l'équation suivante :

$$z_k = \frac{c}{\sqrt{\varepsilon_{r_k}}}\tau_k \quad (5)$$

Pour chaque couche du sol d'indice k, on obtient donc sa profondeur apparente $z_k$ et la permittivité relative moyenne $\varepsilon_{r_k}$ de l'ensemble des couches entre la surface du sol et la couche d'indice k.

Pour obtenir l'épaisseur et la permittivité relative de chaque couche individuelle, on utilise l'équation de Dix :

$$v_{int,n} = \sqrt{\frac{t_n v_{g,n}^2 - t_{n-1} v_{g,n-1}^2}{t_n - t_{n-1}}} \quad (6)$$

$v_{g,n}$ est la vitesse moyenne de propagation du signal depuis la surface du sol jusqu'au fond de la couche d'indice n,

$v_{g,n-1}$ est la vitesse moyenne de propagation du signal depuis la surface du sol jusqu'au fond de la couche d'indice n-1,

$t_n$ est le temps de trajet aller-retour entre la surface du sol et le fond de la couche d'indice n

$t_{n-1}$ est le temps de trajet aller-retour entre la surface du sol et le fond de la couche d'indice n-1

n = 1 correspond à la surface du sol.

$v_{g,n}$, $v_{g,n-1}$ sont calculés à partir de l'équation (2)

$v_{int,n}$ correspond à une vitesse moyenne de propagation du signal à l'intérieur de la couche d'indice n.

Alternativement, l'équation (6) peut être remplacée par l'équation (7) :

$$v_{int,n} = \frac{1}{t_n - t_{n-1}}(t_n v_{g,n} - t_{n-1} v_{g,n-1}) \quad (7)$$

En appliquant l'équation (6) ou (7), on en déduit la valeur de $v_{int,n}$ puis la valeur finale de permittivité relative de la couche n à l'aide de l'équation (2), puis enfin l'épaisseur de la couche avec l'équation :

$$w_n = (t_n - t_{n-1}) \times v_{int,n}, \text{ où } w_n = z_n - z_{n-1}$$

Dans le cas où $t_n v_{g,n}^2 < t_{n-1} v_{g,n-1}^2$, $v_{int,n}$ est une valeur imaginaire. Afin d'éliminer les possibilités d'obtenir une valeur imaginaire, avant d'appliquer l'équation de Dix, à chaque itération, on vérifie si $t_n v_{g,n}^2 > t_{n-1} v_{g,n-1}^2$. Si ce n'est pas le cas, on conserve uniquement les paramètres qui donnent le facteur de corrélation le plus élevé et on supprime

les autres.

On décrit à présent un second mode de réalisation de l'invention qui s'applique avantageusement lorsque l'épaisseur de la première couche est faible en comparaison avec la résolution temporelle du radar qui est liée à sa fréquence porteuse.

Dans une telle situation, on peut considérer que le temps de trajet aller-retour entre la surface du sol et le fond de la première couche est confondu avec le trajet direct simple du signal. Dans ce cas, la courbe de diffraction prend une forme linéaire qui correspond à l'asymptote d'une hyperbole. Un modèle linéaire est alors plus approprié pour estimer les trajets entre la surface du sol et l'interface entre la première couche et la seconde couche.

Dans ce second mode de réalisation de l'invention, la méthode décrite à la figure 1 est modifiée afin d'appliquer certaines étapes spécifiques uniquement pour l'estimation de la première couche.

Les étapes 101 à 108 sont appliquées à l'identique à la différence près que l'équation (1) utilisée pour calculer le retard t est remplacée par l'équation suivante :

$$t = \frac{1}{v_g}\left|x_{Tx_j} + x_{Rx_i} - 2x_{c_p}\right| \quad (7)$$

L'étape 109 consiste tout d'abord à déterminer le chemin qui correspond le plus probablement à la première couche comme étant celui qui présente le facteur de corrélation le plus élevé.

Ensuite, l'épaisseur de la première couche est calculée à l'aide de l'équation (5).

$$w_1 = \frac{c}{\sqrt{\varepsilon_{r_1}}}\tau_1$$

Ce chemin permet donc de définir la permittivité relative $\varepsilon_{r_1}$ et l'épaisseur $w_1$ de la première couche.

Avantageusement, le seuil de détection utilisé à l'étape 107 pour la première couche a une valeur inférieure à celui utilisé pour les couches supérieures du fait que le modèle utilisé pour la première couche est moins précis.

Dans une variante de réalisation du second mode de réalisation, lors du calcul des caractéristiques des couches ultérieures, on compare le coefficient de corrélation pour le trajet obtenu pour la première couche sur la base de l'équation (7) et celui obtenu pour la première couche sur la base de l'équation (1) et on conserve uniquement celui qui présente le coefficient de corrélation le plus élevé.

Dans une autre variante de réalisation de l'invention, les valeurs de permittivité relative obtenues pour chaque couche du sol sont interpolées afin de déterminer une courbe d'évolution de la permittivité relative en fonction de la profondeur du sol ou encore une distribution de probabilité de la permittivité relative en fonction de la profondeur du sol.

Les figures 3a, 3b et 3c donnent des exemples des sorties de certaines étapes intermédiaires de la méthode selon l'invention pour une antenne d'émission.

La figure 3a représente les réponses impulsionnelles obtenues à l'étape 101 pour une antenne d'émission sous la forme d'une matrice donnant les valeurs des amplitudes de trajets mesurés pour différentes antennes en réception, avec un nombre d'antennes en réception N_Rx égal à 8.

La figure 3b représente les résultats obtenus en sortie de l'étape 102 sous la forme de cercles positionnés sur la matrice de la figure 3a pour identifier les trajets multiples détectés

La figure 3c représente un exemple de matrice de corrélation donnant les valeurs des coefficients de corrélation pour chaque couple de retard et permittivité relative.

La figure 4 schématise un dispositif 400 de détermination de caractéristiques diélectriques du sol selon l'invention. Le dispositif 400 comporte au moins un module d'acquisition de signaux radar GPR, par exemple un radar à pénétration de sol multi antennes, et une unité de traitement UT configurée pour exécuter les étapes de mise en œuvre de l'invention à partir des mesures fournies par le radar GPR.

L'unité de traitement UT peut être réalisée sous forme logicielle et/ou matérielle notamment en utilisant un ou plusieurs processeur(s) et une ou plusieurs mémoire(s). Le processeur peut être un processeur générique, un processeur spécifique, un circuit intégré propre à une application (connu aussi sous le nom anglais d'ASIC pour « Application-Specific Integrated Circuit ») ou un réseau de portes programmables in situ (connu aussi sous le nom anglais de FPGA pour « Field-Programmable Gate Array »).

L'invention peut être utilisée conjointement avec un algorithme de détection de cibles radar selon l'état de l'art. En particulier, l'invention peut être utilisée pour détecter des mines ou des canalisations souterraines.

Références

[1] Serkan, S., and V. Borecky. "Estimation methods for obtaining GPR signal velocity." Proceedings of the Third Interlational Conference on ACSEE, Zurich, Switzerland. 2015.
[2] Forte, Emanuele, and Michele Pipan. "Review of multi-offset GPR applications: Data acquisition, processing and analysis." Signal processing 132 (2017): 210-220.
[3] Schneider, William A. "Developments in seismic data processing and analysis (1968-1970)." Geophysics 36.6 (1971): 1043-1073.
[4] Grote, K., S. Hubbard, and Y. Rubin. "GPR monitoring of volumetric water content in soils applied to highway construction and maintenance." The Leading Edge 21.5 (2002): 482-504.
[5] Özdemir, Caner, et al. "A review on migration methods in B-scan ground penetrating radar imaging." Mathematical Problems in Engineering 2014 (2014).
[6] Dong, Zejun, et al. "3D Migration Depth Focus Velocity Analysis of Hand-Held Ground Penetrating Radar." Geosciences 12.4 (2022): 178.
[7] R.-M. Cramer, R. A. Scholtz, and M. Z. Win, "Evaluation of an ultrawide-band propagation channel," IEEE Transactions on Antennas and Propagation, vol. 50, no. 5, pp. 561-570, 2002.
[8] U. Schwarz, "Mathematical-statistical description of the iterative beam removing technique (method clean)," Astronomy and Astrophysics, vol. 65, p. 345, 1978.
[9] A. Walia et al. "Reviewing methods for determination of Dielectric Constant required to Calibrate GPR Study for Asphalt Layers", 2021 IOP Conf. Ser.: Mater. Sci. Eng. 1075 012026, DOI 10.1088/1757-899X/1075/1/012026, XP093059141.

## Revendications

1. Méthode de détermination de la permittivité relative d'un matériau comprenant les étapes de :

    - Acquérir (101) un ensemble de mesures à l'aide d'un radar à pénétration de sol muni de NTx antennes en émission et NRx antennes en réception disposées dans un plan parallèle à la surface du matériau, avec NRx et NTx deux entiers positifs au moins égaux à deux, l'ensemble de mesures étant constitué de réponses impulsionnelles pour chaque couple constitué d'une antenne en émission et d'une antenne en réception,
    - Extraire (102) de chaque réponse impulsionnelle, les composantes relatives à des trajets multiples du signal radar, sous la forme d'un vecteur donnant l'amplitude du signal en fonction de différentes valeurs de retards correspondant à différents trajets,
    - Estimer (103), pour chaque antenne en émission, une coordonnée, dans le plan du radar, de chaque cible associée à un trajet en recherchant la coordonnée de l'antenne en réception qui maximise la réponse impulsionnelle,
    - Calculer (104), pour plusieurs hypothèses de valeurs de permittivité relative du matériau, une estimée d'un retard du signal radar à partir d'un modèle de courbe hyperbolique défini par les caractéristiques du radar et les coordonnées de la cible associée à un trajet du signal,
    - Rechercher (105), dans l'ensemble des composantes extraites des réponses impulsionnelles, pour chaque couple constitué d'une antenne en émission et d'une antenne en réception, au moins une amplitude associée à un retard le plus proche de l'estimée d'un retard calculé à l'étape précédente,
    - Calculer (106), pour chaque antenne en émission, un coefficient de corrélation desdites amplitudes sur l'ensemble des antennes en réception,
    - Itérer les étapes 104 à 106 pour plusieurs valeurs de permittivité relative du matériau,
    - Itérer les étapes 103 à 106 pour toutes les valeurs de retard correspondant à des trajets multiples du signal radar pour obtenir NTx matrices de corrélation donnant, pour chaque couple de valeur de permittivité relative du matériau et valeur de retard, une valeur de coefficient de corrélation,
    - Eliminer (107) les valeurs des matrices de corrélation pour lesquelles le coefficient de corrélation est inférieur à un seuil de détection prédéterminé,
    - Filtrer (108) les valeurs restantes pour ne conserver qu'un vecteur contenant un ensemble de valeurs de retard toutes différentes et une valeur associée de permittivité relative du matériau
    - En déduire (109), pour chaque couche du matériau une estimée de sa permittivité relative et de son épaisseur.

2. Méthode de détermination de la permittivité relative d'un matériau selon la revendication 1 dans laquelle l'étape

d'extraire (102) de chaque réponse impulsionnelle, les composantes relatives à des trajets multiples du signal radar est réalisée au moyen d'un algorithme par haute résolution.

3. Méthode de détermination de la permittivité relative d'un matériau selon l'une quelconque des revendications précédentes dans laquelle l'étape (104) de calcul d'une estimée t d'un retard du signal radar est réalisée au moyen de l'équation hyperbolique suivante :

$$t = \frac{1}{v_g}\left[\sqrt{\left(x_{Tx_j} - x_{c_p}\right)^2 + \left(\frac{\Delta y}{2}\right)^2 + \left(\frac{v_g \tau_p}{2}\right)^2} + \sqrt{\left(x_{Rx_i} - x_{c_p}\right)^2 + \left(\frac{\Delta y}{2}\right)^2 + \left(\frac{v_g \tau_p}{2}\right)^2}\right]$$

Avec $x_{cp}$ la coordonnée de la cible estimée à l'étape 103, $x_{Tx_j}$ la coordonnée de chaque antenne en émission, $x_{Rx_i}$ la coordonnée de chaque antenne en réception, $\Delta y$ la distance entre une antenne en émission et une antenne en réception, $v_g$ la vitesse moyenne de propagation du signal dans le matériau, $\tau_p$ la valeur d'un retard estimée à l'étape 102.

4. Méthode de détermination de la permittivité relative d'un matériau selon l'une quelconque des revendications précédentes dans laquelle l'étape (105) est réalisée en :

- Recherchant, pour chaque couple constitué d'une antenne en émission et d'une antenne en réception, l'amplitude $a_{ip}$ associée au retard le plus proche de l'estimée d'un retard calculé à l'étape 104,
- Définir une fenêtre temporelle de N+1 échantillons, N étant un entier positif au moins égal à 1, la fenêtre étant centrée sur l'amplitude sélectionnée à l'étape précédente et sélectionner N/2 valeurs d'amplitudes correspondants aux valeurs de retard comprises dans la fenêtre temporelle.

5. Méthode de détermination de la permittivité relative d'un matériau selon la revendication 4 dans laquelle le coefficient de corrélation est calculé (106) au moyen de la relation suivante :

$$R(t, \varepsilon_r) = \frac{\sum_{p_j=p-N/2}^{p+N/2}\left[\sum_{i=1}^{NRx} a_{ip_j}\right]^2}{M\sum_{p_j=p-N/2}^{p+N/2}\sum_{i=1}^{NRx} a_{ip_j}^2}$$

avec $a_{ip}$ l'amplitude associée au retard le plus proche de l'estimée d'un retard calculé à l'étape 104, p est l'indice dudit retard et $a_{ip_j}$ est l'amplitude correspondant à l'indice temporel $p_j$ dans la dite fenêtre temporelle, $\varepsilon_r$ est l'hypothèse de valeur de permittivité relative.

6. Méthode de détermination de la permittivité relative d'un matériau selon l'une quelconque des revendications précédentes dans laquelle la valeur du seuil est comprise dans l'intervalle ] 0.5 ; 1 [

7. Méthode de détermination de la permittivité relative d'un matériau selon l'une quelconque des revendications précédentes dans laquelle l'étape de filtrer (108) les valeurs restantes comporte les sous étapes de :

- Sélectionner les couples qui présentent sensiblement les mêmes valeurs de retard pour toutes les antennes en émission,
- Ordonner les couples selon la valeur des retards,
- Lorsque plusieurs valeurs de permittivité relative consécutives sont sensiblement égales, conserver uniquement le couple ayant la valeur de retard la plus élevée.

8. Méthode de détermination de la permittivité relative d'un matériau selon la revendication 7 dans laquelle, lorsque plusieurs couples présentent sensiblement les mêmes valeurs de retard pour toutes les antennes en émission mais des valeurs de permittivité relative différentes, on conserve uniquement la valeur de permittivité relative qui maximise le coefficient de corrélation.

9. Méthode de détermination de la permittivité relative d'un matériau selon l'une quelconque des revendications précédentes dans laquelle l'étape de déduire (109), pour chaque couche du matériau, une estimée de sa permittivité

relative et de son épaisseur comporte les sous étapes de :

- Estimer la profondeur apparente de chaque couche à partir de chaque couple de valeurs de retard et de permittivité relative,
- Déterminer itérativement la permittivité relative de chaque couche en appliquant l'équation de Dix aux valeurs de retard et de vitesse de propagation, dérivée de la valeur de permittivité relative,
- Déterminer l'épaisseur de chaque couche à partir des valeurs de retard et de vitesse de propagation.

**10.** Méthode de détermination de la permittivité relative d'un matériau selon la revendication 9 dans laquelle, au cours de l'étape de déterminer itérativement la permittivité relative de chaque couche, lorsque deux trajets consécutifs conduisent à obtenir une vitesse de propagation imaginaire, le trajet, parmi les deux trajets, ayant le facteur de corrélation le plus faible est éliminé.

**11.** Méthode de détermination de la permittivité relative d'un matériau selon l'une quelconque des revendications précédentes, dans lequel le matériau est multi-couches et la méthode comprend une phase préalable d'estimation de la permittivité relative de la première couche du matériau pour laquelle le modèle de courbe hyperbolique pour la première couche du matériau est un modèle asymptotique linéaire et la phase préalable comprend :

- une étape de détermination du trajet correspondant à la première couche comme celui qui maximise le coefficient de corrélation,
- la sélection de la permittivité relative associée audit trajet comme correspondant à la première couche,
- le calcul de l'épaisseur de la première couche à partir des valeurs de retard et permittivité relative associées au trajet.

**12.** Méthode de détermination de la permittivité relative d'un matériau selon la revendication 11 dans laquelle le seuil de détection utilisé pour la première couche lors de la phase préalable a une valeur inférieure au seuil de détection utilisé pour les couches ultérieures.

**13.** Méthode de détermination de la permittivité relative d'un matériau selon l'une quelconque des revendications précédentes dans laquelle le matériau est un sol.

**14.** Dispositif pour déterminer la permittivité relative d'un matériau comprenant un radar à pénétration de sol muni de NTx antennes en émission et NRx antennes en réception et **caractérisé par** une unité de traitement configurés pour mettre en œuvre les étapes de la méthode selon l'une quelconque des revendications précédentes.

**15.** Programme d'ordinateur comprenant des instructions de code qui conduisent le dispositif selon la revendication 14 à exécuter les étapes de la méthode selon l'une quelconque des revendications 1 à 13.

**16.** Support lisible par ordinateur, sur lequel est enregistré le programme d'ordinateur selon la revendication 15.

## Patentansprüche

**1.** Verfahren zum Bestimmen der relativen Permittivität eines Materials, umfassend die folgenden Schritte:

- Erfassen (101) eines Satzes von Messungen mit Hilfe eines Bodenradars, der mit NTx Sendeantennen und NRx Empfangsantennen versehen ist, die in einer Ebene parallel zur Oberfläche des Materials angeordnet sind, wobei NRx und NTx zwei positive ganze Zahlen mindestens gleich zwei sind, wobei der Satz von Messungen aus Impulsantworten für jedes Paar besteht, das aus einer Sendeantenne und einer Empfangsantenne besteht,
- Extrahieren (102), aus jeder Impulsantwort, der Komponenten betreffend Mehrfachflusswege des Radarsignals in Form eines Vektors, der die Amplitude des Signals in Abhängigkeit von verschiedenen Verzögerungswerten entsprechend verschiedenen Flusswegen angibt,
- Schätzen (103), für jede Sendeantenne, einer Koordinate in der Ebene des Radars von jedem Ziel, das einem Flussweg zugeordnet ist, durch Suchen der Koordinate der Empfangsantenne, die die Impulsantwort maximiert,
- Berechnen (104), für mehrere angenommene relative Permittivitätswerte des Materials, einer Schätzung einer Verzögerung des Radarsignals anhand eines hyperbolischen Kurvenmodells, das durch die Kennzeichen des Radars und die Koordinaten des einem Flussweg des Signals zugeordneten Ziels definiert ist,
- Suchen (105), in dem Satz der aus den Impulsantworten extrahierten Komponenten, für jedes aus einer

Sendeantenne und einer Empfangsantenne bestehende Paar, mindestens einer Amplitude, die einer Verzögerung zugeordnet ist, die der Schätzung einer im vorhergehenden Schritt berechneten Verzögerung am nächsten kommt,
- Berechnen (106), für jede Sendeantenne, eines Korrelationskoeffizienten der Amplituden an dem Satz der Empfangsantennen,
- Wiederholen der Schritte 104 bis 106 für mehrere relative Permittivitätswerte des Materials,
- Wiederholen der Schritte 103 bis 106 für alle Verzögerungswerte entsprechend Mehrfachflusswegen des Radarsignals, um NTx Korrelationsmatrizen zu erhalten, die für jedes Paar aus dem relativen Permittivitätswert des Materials und dem Verzögerungswert einen Korrelationskoeffizientenwert angibt,
- Eliminieren (107) der Werte der Korrelationsmatrizen, für die der Korrelationskoeffizient kleiner als ein vorbestimmter Erkennungsschwellenwert ist,
- Filtern (108) der verbleibenden Werte, um nur einen Vektor zu behalten, der einen Satz von jeweils unterschiedlichen Verzögerungswerten und einen zugeordneten relativen Permittivitätswert des Materials enthält,
- Abziehen von diesem (109), für jede Materialschicht, einer Schätzung ihrer relativen Permittivität und ihrer Dicke.

2. Verfahren zum Bestimmen der relativen Permittivität eines Materials nach Anspruch 1, wobei der Schritt des Extrahierens (102), aus jeder Impulsantwort, der Komponenten betreffend Mehrfachflusswege des Radarsignals mittels eines hochauflösenden Algorithmus durchgeführt wird.

3. Verfahren zum Bestimmen der relativen Permittivität eines Materials nach einem der vorhergehenden Ansprüche, wobei der Schritt (104) des Berechnens einer Schätzung t einer Verzögerung des Radarsignals mittels der folgenden hyperbolischen Gleichung durchgeführt wird:

$$t = \frac{1}{v_g}\left[\sqrt{\left(x_{Tx_j} - x_{c_p}\right)^2 + \left(\frac{\Delta y}{2}\right)^2 + \left(\frac{v_g \tau_p}{2}\right)^2} + \sqrt{\left(x_{Rx_i} - x_{c_p}\right)^2 + \left(\frac{\Delta y}{2}\right)^2 + \left(\frac{v_g \tau_p}{2}\right)^2}\right]$$

wobei $x_{c_p}$ die Koordinate des im Schritt 103 geschätzten Ziels ist, $x_{Tx_j}$ die Koordinate jeder Sendeantenne ist, $x_{Rx_i}$ die Koordinate jeder Empfangsantenne ist, $\Delta y$ der Abstand zwischen einer Sendeantenne und einer Empfangsantenne ist, $v_g$ die mittlere Ausbreitungsgeschwindigkeit des Signals in dem Material ist, $\tau_p$ der Wert einer in Schritt 102 geschätzten Verzögerung ist.

4. Verfahren zum Bestimmen der relativen Permittivität eines Materials nach einem der vorhergehenden Ansprüche, wobei der Schritt (105) durchgeführt wird durch:

- Suchen, für jedes aus einer Sendeantenne und einer Empfangsantenne bestehende Paar, der Amplitude $a_{ip}$, die der Verzögerung zugeordnet ist, die der Schätzung einer im Schritt 104 berechneten Verzögerung am nächsten kommt,
- Definieren eines Zeitfensters von N+1 Abtastungswerten, wobei N eine positive ganze Zahl mindestens gleich 1 ist, wobei das Fenster auf die im vorhergehenden Schritt ausgewählte Amplitude zentriert ist, und Auswählen von N/2 Amplitudenwerten entsprechend den in dem Zeitfenster enthaltenen Verzögerungswerten.

5. Verfahren zum Bestimmen der relativen Permittivität eines Materials nach Anspruch 4, wobei der Korrelationskoeffizient mittels der folgenden Beziehung berechnet (106) wird:

$$R(t, \varepsilon_r) = \frac{\sum_{p_j=p-N/2}^{p+N/2}\left[\sum_{i=1}^{NRx} a_{ip_j}\right]^2}{M\sum_{p_j=p-N/2}^{p+N/2}\sum_{i=1}^{NRx} a_{ip_j}^2}$$

wobei $a_{ip}$ die Amplitude ist, die der Verzögerung zugeordnet ist, die der Schätzung einer im Schritt 104 berechneten Verzögerung am nächsten kommt, p der Index der Verzögerung ist und $a_{ip_j}$ die Amplitude ist, die dem Zeitindex $p_j$ in dem Zeitfenster entspricht, $\varepsilon_r$ der angenommene relative Permittivitätswert ist.

**6.** Verfahren zum Bestimmen der relativen Permittivität eines Materials nach einem der vorhergehenden Ansprüche, wobei der Schwellenwert zwischen dem Intervall ] 0,5; 1 [ liegt.

**7.** Verfahren zum Bestimmen der relativen Permittivität eines Materials nach einem der vorhergehenden Ansprüche, wobei der Schritt zum Filtern (108) der verbleibenden Werte die folgenden Unterschritte aufweist:

- Auswählen der Paare, die im Wesentlichen die gleichen Verzögerungswerte für alle Sendeantennen aufweisen,
- Ordnen der Paare gemäß dem Wert der Verzögerungen,
- wenn mehrere aufeinanderfolgende relative Permittivitätswerte im Wesentlichen gleich sind, Behalten nur des Paars mit dem höchsten Verzögerungswert.

**8.** Verfahren zum Bestimmen der relativen Permittivität eines Materials nach Anspruch 7, wobei, wenn mehrere Paare im Wesentlichen die gleichen Verzögerungswerte für alle Sendeantennen, jedoch verschiedene relative Permittivitätswerte aufweisen, nur der relative Permittivitätswert behalten wird, der den Korrelationskoeffizienten maximiert.

**9.** Verfahren zum Bestimmen der relativen Permittivität eines Materials nach einem der vorhergehenden Ansprüche, wobei der Schritt zum Abziehen (109), für jede Materialschicht, einer Schätzung ihrer relativen Permittivität und ihrer Dicke, die folgenden Unterschritte aufweist:

- Schätzen der scheinbaren Tiefe jeder Schicht anhand jedes Paares von Verzögerungs- und relativen Permittivitätswerten,
- wiederholtes Bestimmen der relativen Permittivität jeder Schicht durch Anwenden der Dix-Gleichung auf die Verzögerungs- und Ausbreitungsgeschwindigkeitswerte, die von dem relativen Permittivitätswert hergeleitet ist,
- Bestimmen der Dicke jeder Schicht anhand der Verzögerungs- und Ausbreitungsgeschwindigkeitswerte.

**10.** Verfahren zum Bestimmen der relativen Permittivität eines Materials nach Anspruch 9, wobei im Verlauf des Schritts zum wiederholten Bestimmen der relativen Permittivität jeder Schicht, wenn zwei aufeinanderfolgende Flusswege dazu führen, dass eine imaginäre Ausbreitungsgeschwindigkeit erhalten wird, der Flussweg von den zwei Flusswegen mit dem geringsten Korrelationsfaktor eliminiert wird.

**11.** Verfahren zum Bestimmen der relativen Permittivität eines Materials nach einem der vorhergehenden Ansprüche, wobei das Material mehrschichtig ist und das Verfahren eine vorherige Phase zum Schätzen der relativen Permittivität der ersten Schicht des Materials umfasst, für die das hyperbolische Kurvenmodell für die erste Schicht des Materials ein lineares asymptotisches Modell ist, und die vorherige Phase Folgendes umfasst:

- einen Schritt zum Bestimmen des der ersten Schicht entsprechenden Flusswegs als denjenigen, der den Korrelationskoeffizienten maximiert,
- Auswählen der dem Flussweg als der ersten Schicht entsprechend zugeordneten relativen Permittivität,
- Berechnen der Dicke der ersten Schicht anhand der Verzögerungs- und relativen Permittivitätswerte, die dem Flussweg zugeordnet sind.

**12.** Verfahren zum Bestimmen der relativen Permittivität eines Materials nach Anspruch 11, wobei die Erkennungsschwelle, die für die erste Schicht bei der vorherigen Phase verwendet wird, einen Wert aufweist, der kleiner als die Erkennungsschwelle ist, die für die folgenden Schichten verwendet wird.

**13.** Verfahren zum Bestimmen der relativen Permittivität eines Materials nach einem der vorhergehenden Ansprüche, wobei das Material ein Boden ist.

**14.** Vorrichtung zum Bestimmen der relativen Permittivität eines Materials, umfassend einen Bodenradar, der mit NTx Sendeantennen und NRx Empfangsantennen versehen und durch eine Behandlungseinheit gekennzeichnet ist, die dafür konfiguriert ist, die Schritte des Verfahrens nach einem der vorhergehenden Ansprüche umzusetzen.

**15.** Computerprogramm, umfassend Codeanweisungen, die die Vorrichtung nach Anspruch 14 veranlassen, die Schritte des Verfahrens nach einem der Ansprüche 1 bis 13 auszuführen.

**16.** Computerlesbares Medium, auf dem das Computerprogramm nach Anspruch 15 gespeichert ist.

## Claims

1. A method for determining the relative permittivity of a material, comprising the steps of:

    - acquiring (101) a set of measurements using a ground-penetrating radar provided with NTx transmitting antennas and NRx receiving antennas disposed in a plane parallel to the surface of the material, with NTx and NRx being two positive integers at least equal to two, the set of measurements being made up of pulse responses for each pair constituted by a transmitting antenna and a receiving antenna;
    - extracting (102), from each pulse response, the components relating to multiple paths of the radar signal, in the form of a vector providing the amplitude of the signal as a function of different delay values corresponding to different paths;
    - estimating (103), for each transmitting antenna, a coordinate, in the plane of the radar, of each target associated with a path by finding the coordinate of the receiving antenna that maximises the pulse response;
    - computing (104), for several assumed relative permittivity values of the material, an estimate of a delay of the radar signal from a hyperbolic curve model defined by the features of the radar and the coordinates of the target associated with a path of the signal;
    - finding (105), in all the components extracted from the pulse responses, for each pair made up of a transmitting antenna and a receiving antenna, at least one amplitude associated with a delay closest to the estimate of a delay computed in the previous step;
    - computing (106), for each transmitting antenna, a correlation coefficient for said amplitudes on all the receiving antennas;
    - iterating steps 104 to 106 for a several relative permittivity values of the material;
    - iterating steps 103 to 106 for all the delay values corresponding to multiple paths of the radar signal in order to obtain NTx correlation matrices providing, for each relative permittivity value of the material and delay value pair, a correlation coefficient value;
    - eliminating (107) the values of the correlation matrices for which the correlation coefficient is less than a predetermined detection threshold;
    - filtering (108) the remaining values so as to retain only one vector containing a set of delay values that are all different and an associated relative permittivity value of the material;
    - deducing (109) therefrom, for each layer of the material, an estimate of its relative permittivity and its thickness.

2. The method for determining the relative permittivity of a material according to claim 1, wherein the step (102) of extracting, from each pulse response, the components relating to multiple paths of the radar signal is carried out by means of a high-resolution algorithm.

3. The method for determining the relative permittivity of a material according to any one of the preceding claims, wherein the step (104) of computing an estimate t of a delay of the radar signal is carried out by means of the following hyperbolic equation:

$$t = \frac{1}{v_g}\left[\sqrt{\left(x_{Tx_j} - x_{c_p}\right)^2 + \left(\frac{\Delta y}{2}\right)^2 + \left(\frac{v_g \tau_p}{2}\right)^2} + \sqrt{\left(x_{Rx_i} - x_{c_p}\right)^2 + \left(\frac{\Delta y}{2}\right)^2 + \left(\frac{v_g \tau_p}{2}\right)^2}\right]$$

with $x_{c_p}$ being the coordinate of the target estimated in step 103, $x_{Tx_j}$ being the coordinate of each transmitting antenna, $x_{Rx_i}$ being the coordinate of each receiving antenna, $\Delta y$ being the distance between a transmitting antenna and a receiving antenna, $v_g$ being the mean propagation velocity of the signal in the material and $\tau_p$ being the value of a delay estimated in step 102.

4. The method for determining the relative permittivity of a material according to any one of the preceding claims, wherein step (105) is carried out by:

    - finding, for each pair made up of a transmitting antenna and a receiving antenna, the amplitude $a_{ip}$ associated with the delay closest to the estimate of a delay computed in step 104;
    - defining a time window of N+1 samples, N being a positive integer at least equal to 1, the window being centred on the amplitude selected in the preceding step, and selecting N/2 amplitude values corresponding to the delay values contained in the time window.

**5.** The method for determining the relative permittivity of a material according to claim 4, wherein the correlation coefficient is computed (106) by means of the following relation:

$$R(t,\varepsilon_r) = \frac{\sum_{p_j=p-N/2}^{p+N/2}\left[\sum_{i=1}^{NRx} a_{ip_j}\right]^2}{M\sum_{p_j=p-N/2}^{p+N/2}\sum_{i=1}^{NRx} a_{ip_j}^2}$$

with $a_{ip}$ being the amplitude associated with the delay closest to the estimate of a delay computed in step 104, p being the index of said delay and $a_{ip_j}$ being the amplitude corresponding to the time index $p_j$ in said time window, with $\varepsilon_r$ being the assumed relative permittivity value.

**6.** The method for determining the relative permittivity of a material according to any one of the preceding claims, wherein the value of the threshold is comprised in the interval ] 0.5; 1 [.

**7.** The method for determining the relative permittivity of a material according to any one of the preceding claims, wherein the step (108) of filtering the remaining values comprises the sub-steps of:

- selecting the pairs that have substantially the same delay values for all the transmitting antennas;
- ordering the pairs according to the value of the delays;
- retaining, when several consecutive relative permittivity values are substantially equal, only the pair with the highest delay value.

**8.** The method for determining the relative permittivity of a material according to claim 7, wherein, when several pairs have substantially the same delay values for all the transmitting antennas but different relative permittivity values, only the relative permittivity value that maximises the correlation coefficient is retained.

**9.** The method for determining the relative permittivity of a material according to any one of the preceding claims, wherein the step (109) of deducing, for each layer of the material, an estimate of its relative permittivity and its thickness, comprises the sub-steps of:

- estimating the apparent depth of each layer from each pair of delay and relative permittivity values;
- iteratively determining the relative permittivity of each layer by applying the Dix equation to the delay and propagation velocity values, derived from the relative permittivity value;
- determining the thickness of each layer from the delay and propagation velocity values.

**10.** The method for determining the relative permittivity of a material according to claim 9, wherein, during the step of iteratively determining the relative permittivity of each layer, when two consecutive paths result in an imaginary propagation velocity being obtained, the path with the lowest correlation factor among the two paths is eliminated.

**11.** The method for determining the relative permittivity of a material according to any one of the preceding claims, wherein the material is multi-layer and the method comprises a prior phase of estimating the relative permittivity of the first layer of the material for which the hyperbolic curve model for the first layer of the material is a linear asymptotic model and the prior phase comprises:

- a step of determining the path corresponding to the first layer as the one that maximises the correlation coefficient;
- selecting the relative permittivity associated with said path as corresponding to the first layer;
- computing the thickness of the first layer from the delay and relative permittivity values associated with the path.

**12.** The method for determining the relative permittivity of a material according to claim 11, wherein the detection threshold used for the first layer during the prior phase has a lower value than the detection threshold used for the subsequent layers.

**13.** The method for determining the relative permittivity of a material according to any one of the preceding claims, wherein the material is the ground.

**14.** A device for determining the relative permittivity of a material comprising a ground-penetrating radar provided with NTx transmitting antennas and NRx receiving antennas and being **characterised by** a processing unit configured to implement the steps of the method according to any one of the preceding claims.

**15.** A computer program comprising code instructions that cause the device according to claim 14 to execute the steps of the method according to any one of claims 1 to 13.

**16.** A computer-readable medium that stores the computer program according to claim 15.

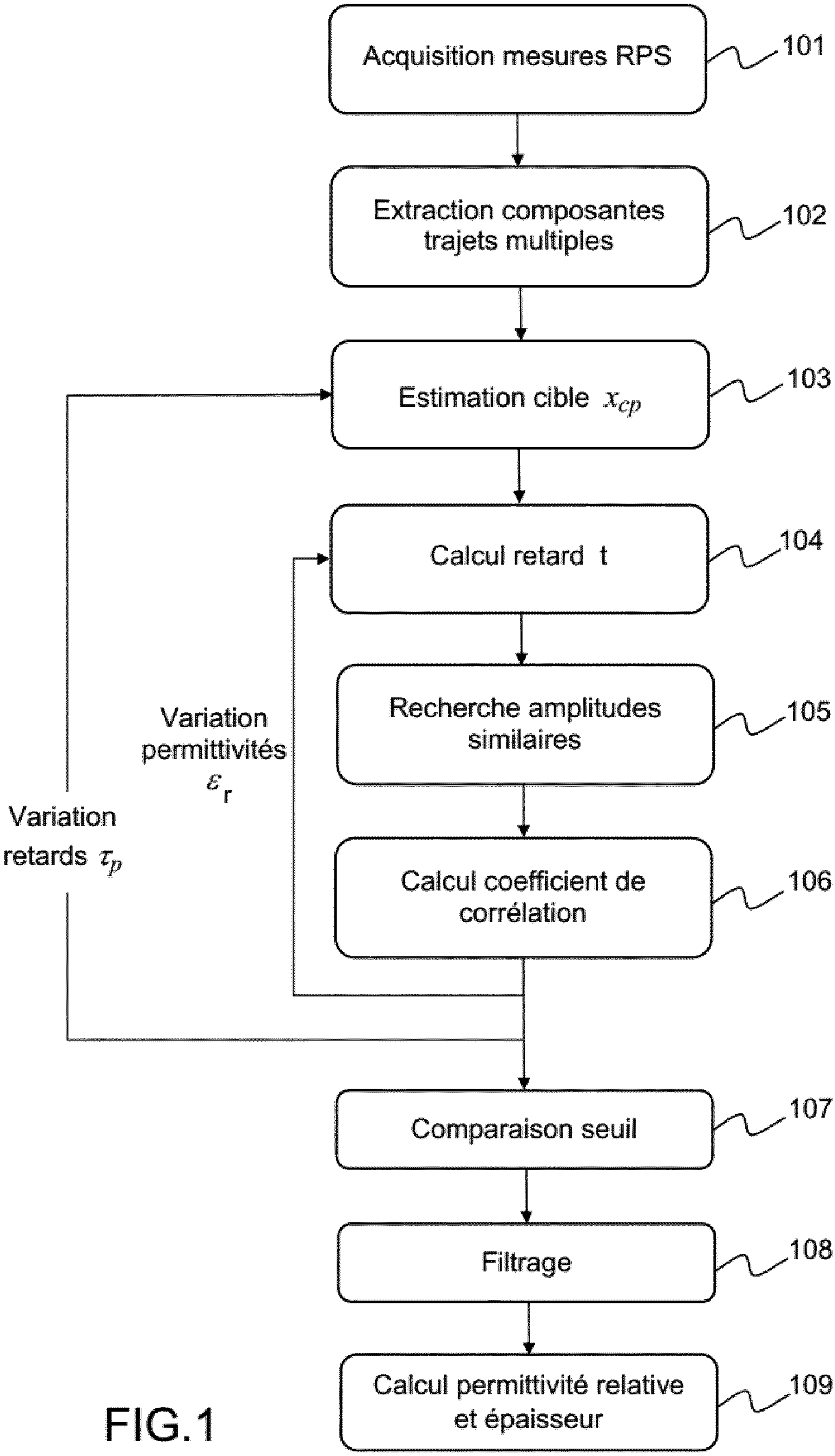
Acquisition mesures RPS
101
Extraction composantes trajets multiples
102
Estimation cible $x_{cp}$
103
Calcul retard t
104
Recherche amplitudes similaires
105
Calcul coefficient de corrélation
106
Variation permittivités $\varepsilon_r$
Variation retards $\tau_p$
Comparaison seuil
107
Filtrage
108
Calcul permittivité relative et épaisseur
109

FIG.1

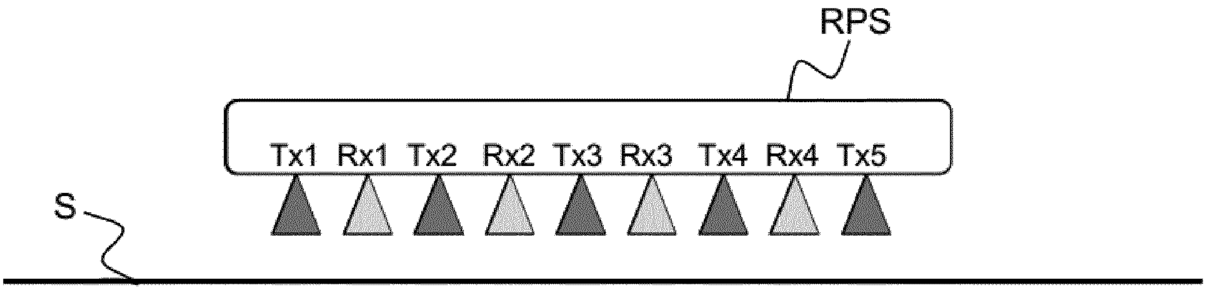
RPS
Tx1 Rx1 Tx2 Rx2 Tx3 Rx3 Tx4 Rx4 Tx5
S

FIG.2a

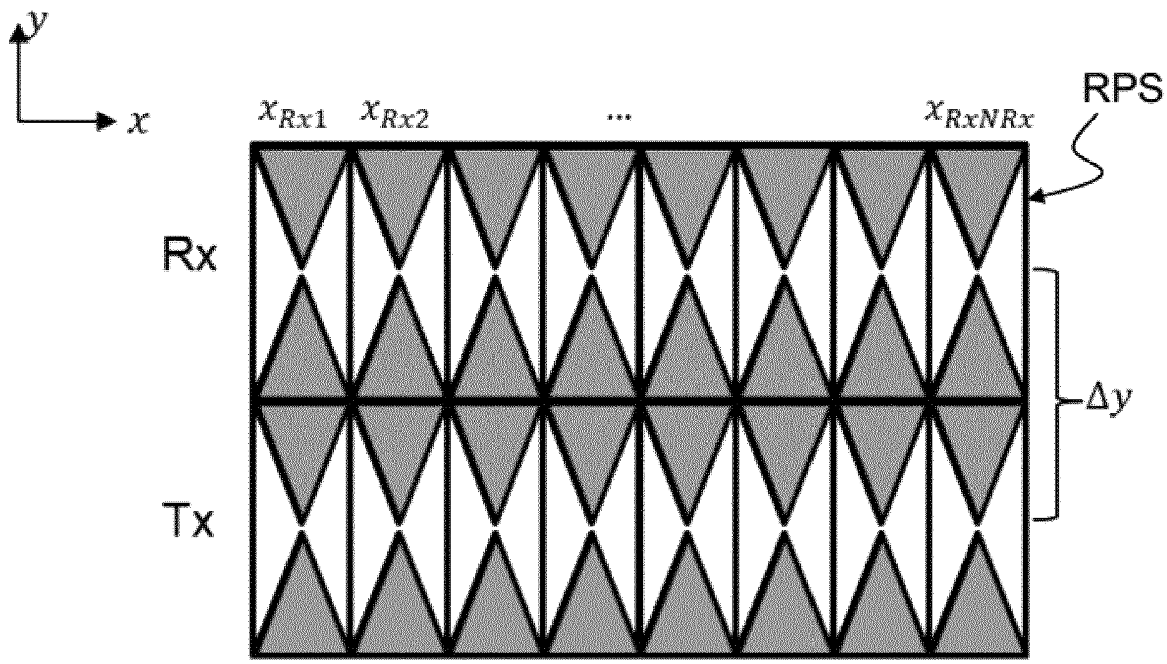
y
x
$x_{Rx1}$ $x_{Rx2}$ ... $x_{RxNRx}$
RPS
Rx
Tx
$\Delta y$

FIG.2b

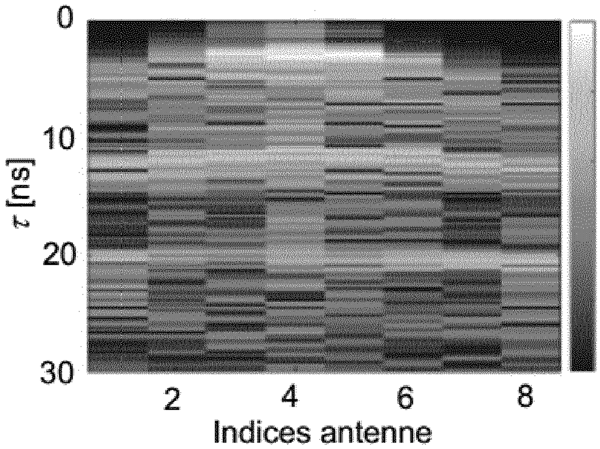


FIG.3a

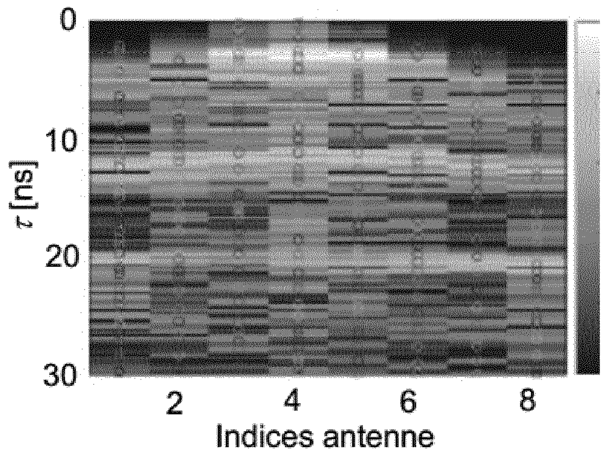


FIG.3b

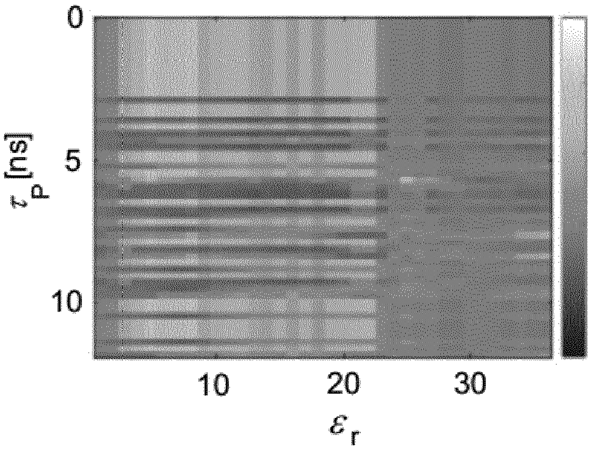


FIG.3c

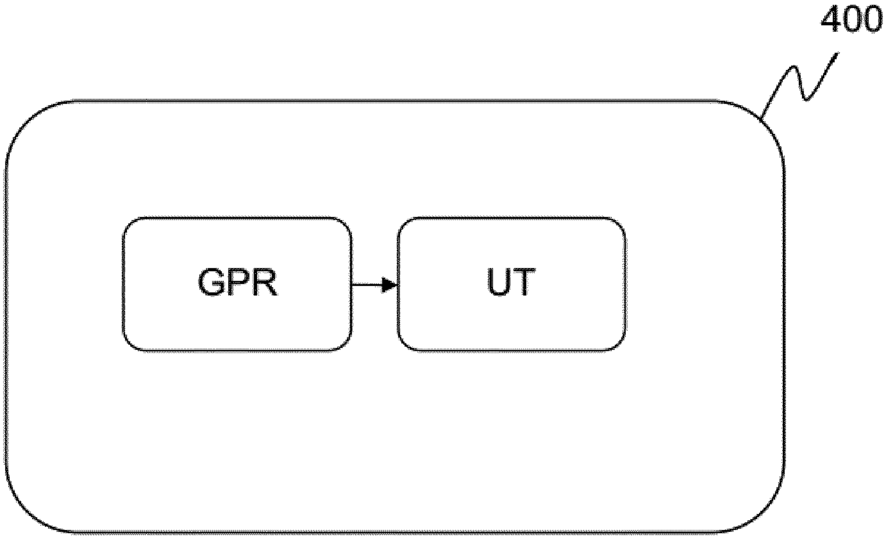


FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **SERKAN, S** ; **V. BORECKY**. Estimation methods for obtaining GPR signal velocity.. *Proceedings of the Third Interlational Conference on ACSEE*, 2015
- **FORTE, EMANUELE** ; **MICHELE PIPAN**. Review of multi-offset GPR applications: Data acquisition, processing and analysis.. *Signal processing*, 2017, vol. 132, 210-220
- **SCHNEIDER, WILLIAM A.** Developments in seismic data processing and analysis (1968-1970).. *Geophysics*, 1971, vol. 36 (6), 1043-1073
- **GROTE, K.** ; **S. HUBBARD** ; **Y. RUBIN**. GPR monitoring of volumetric water content in soils applied to highway construction and maintenance. *The Leading Edge 21.5*, 2002, 482-504
- **ÖZDEMIR, CANER et al.** A review on migration methods in B-scan ground penetrating radar imaging.. *Mathematical Problems in Engineering*, 2014, vol. 2014
- **DONG, ZEJUN et al.** 3D Migration Depth Focus Velocity Analysis of Hand-Held Ground Penetrating Radar.. *Geosciences*, 2022, vol. 12 (4), 178
- **R.-M. CRAMER** ; **R. A. SCHOLTZ** ; **M. Z. WIN**. Evaluation of an ultrawide-band propagation channel. *IEEE Transactions on Antennas and Propagation*, 2002, vol. 50 (5), 561-570
- **U. SCHWARZ**. Mathematical-statistical description of the iterative beam removing technique (method clean). *Astronomy and Astrophysics*, 1978, vol. 65, 345
- **A. WALIA et al.** Reviewing methods for determination of Dielectric Constant required to Calibrate GPR Study for Asphalt Layers. *IOP Conf. Ser.: Mater. Sci. Eng. 1075 012026*, 2021